# EUROPÄISCHE PATENTSCHRIFT

(11) **EP 0 623 018 B1**
(45) Veröffentlichungstag und Bekanntmachung des Hinweises auf die Patenterteilung: **27.12.1995**
(21) Anmeldenummer: 93901686.1
(22) Anmeldetag: 18.01.1993
(51) Int. Cl.: A61K 9/48, A61K 9/50

(54) **VERFAHREN ZUR HERSTELLUNG VON WEICHGELATINEKAPSELN NACH EINEM TROPFVERFAHREN**
PROCESS FOR THE MANUFACTURE OF SOFT GELATIN CAPSULES BY A DRIP-FEED METHOD
PROCEDE DE FABRICATION DE CAPSULES DE GELATINE MOLLE SELON UNE METHODE EN GOUTTE

(30) Priorität: 17.01.1992 DE 4201178; 30.04.1992 US 876863
(43) Veröffentlichungstag der Anmeldung: 09.11.1994
(73) Patentinhaber: ALFATEC-PHARMA GmbH, 69120 Heidelberg (DE)
(72) Erfinder: WUNDERLICH, Jens-Christian, D-6900 Heidelberg (DE); SCHICK, Ursula, D-69198 Schriesheim (DE); WERRY, Jürgen, D-6700 Ludwigshafen (DE); FREIDENREICH, Jürgen, D-6905 Schriesheim (DE)
(74) Vertreter: KUHNEN, WACKER & PARTNER
(86) Internationale Anmeldenummer: DE9300035
(87) Internationale Veröffentlichungsnummer: WO9313761

(56) Entgegenhaltungen:
- FR-A- 1 080 822
- CHEMICAL ABSTRACTS, vol. 113, no. 13, 1990, Columbus, Ohio, US; abstract no. 114127d
- P.H. LIST ET AL. 'HAGERS HANDBUCH DER PHARMAZEUTISCHEN PRAXIS' 1971 , SPRINGER- VERLAG , BERLIN.HEIDELBERG.NEW YORK

## Beschreibung

Die Erfindung betrifft ein Verfahren zur Herstellung von Weichgelatinekapseln nach einem Tropfverfahren, wobei man das pastöse oder flüssige Füllgut mit einer Weichgelatinemasse umhüllt und zur Verfestigung der Gelatinemasse in ein Kühlbad einbringt.

Die Herstellung von Weichgelatinekapseln erfolgt heute vorzugsweise nach einem Stanzverfahren, bei dem die Kapselwand aus zwei Gelatinehälften zusammengesetzt und geformt wird, die aus einem Gelatineband ausgestanzt sind. Vorzugsweise wird das nach der Rotary-Die-Methode arbeitende Scherer-Verfahren benutzt. Hierbei laufen zwei endlose Gelatinebänder gegen zwei nebeneinanderliegende und gegeneinanderlaufende Formwalzen. Während die Gelatinebänder in die Form gepreßt werden und so die Kapselhälften bilden, gelangt über einen exakt dosierenden Füllkeil das fließfähige Füllgut in die entstehende Kapsel. Es folgt das verschweißen der Kapselhälften, das Ausstanzen, ein Waschvorgang zur Befreiung von anhaftendem Oel, ein Rotationstrocknerdurchlauf sowie eine abschließende Hordentrocknung.

Die Rotary-Die-Methode ermöglicht die Herstellung und Befüllung der Kapseln in einem Arbeitsgang und bringt Stundenleistungen bis zu 100.000 Stück. Ein typisches Kennzeichen der Scherer-Kapseln ist die zentrale Schweißnaht in Längsrichtung. Das Scherer-Verfahren besitzt u. a. die folgenden Nachteile:
a) Zur Herstellung der Weichgelatinekapseln kann nur Gelatine eingesetzt werden, deren Qualität nur in engen Grenzen variiert werden kann. Zum Beispiel müssen die folgenden Spezifikationen erfüllt werden: Die Gelatinesorten müssen Gallertfestigkeiten von durchschnittlich 100-200 Bloom sowie Viskositätswerte aufweisen, die über einen längeren Zeitraum thermostabil bleiben, denn beim Ausgießen der Gelatinebänder muß stets eine gleichmäßige Banddicke gewährleistet sein. So dürfen die Viskositäten der eingesetzten Gelatinesorten nach mehrtägiger Belastung bei 60°C nur um maximal 10-15% absinken.
b) Die Formwalzen zum Formen der Gelatinekapseln aus den beiden Gelatinebändern müssen sehr präzise gefertigt sein und sehr genau arbeiten. Sie sind daher in der Herstellung teuer und im Betrieb störungsanfällig.
c) Für die Herstellung von Weichgelatinekapseln ist eine Klimatisierung von 20 bis 30% relativer Feuchtigkeit bei 22° C erforderlich, wie man aus der Adsorptionsisotherme von Wasser an Gelatine-Kapsel-Material entnimmt. Daher müssen alle Herstellungs- und verpackungsräume voll klimatisiert werden.
d) Die Technik der Weichgelatinekapselherstellung erfordert außer den genannten hohen Anforderungen an das Material und die Klimatisierung soviel Know-How, daß sie nur von darauf spezialisierten Herstellern beherrscht wird.
e) Ein weiterer Nachteil besteht darin, daß die beim Stanzverfahren zurückbleibenden Abfälle der Gelatinebänder, die sog. Netzabfälle, nur zu einem geringen Teil (max. 5%) wiederverwendet werden können und daher bis zu 60% der eingesetzten Gelatinemasse entsorgt werden müssen. Diese Netzabfälle sind nämlich herstellungsbedingt mit Trennölen verunreinigt, und bei stark wirksamen Substanzen als Füllgut kann eine Kontamination des ausgestanzten Netzes nicht vermieden werden. Deshalb sind solche Netzabfälle als Sondermüll zu behandeln. Weiterhin enthalten diese Abfälle häufig Farbpigmente, die eine Rückgewinnung der Ausgangsmasse unmöglich machen.
f) Durch das Herstellungsverfahren bedingt ist die fertige Kapsel mit störendem Trennöl behaftet, das durch wirksame Lipoidlösungsmittel wie Tetrachlorethylen, Methylenchlorid usw. entfernt werden muß. Dieser Verfahrensschritt erfordert einen besonderen technischen und kapitalintensiven Aufwand, um jede Kontamination der Weichgelatinekapseln und der Abluft mit gesundheitsschädlichen Lösungsmitteln zu vermeiden. Außerdem ist die Behandlung von Arzneistoffen enthaltenden Weichgelatinekapseln mit solchen gesundheitsschädlichen Lösungsmitteln auch deshalb problematisch, weil die Verbraucher der Weichgelatinekapseln vor der Einnahme der so behandelten Weichgelatinekapseln zurückschrecken könnten.

Ein weiteres Verfahren zur Herstellung von Weichgelatinekapseln nach dem Stand der Technik ist das Tropf- und Blasverfahren, nach der Entwicklerin auch Globex-Verfahren genannt. Hierbei tropft man das lipophile Füllgut aus einer Düse, während gleichzeitig warme Gelatinelösung aus einem die Düse mantelförmig umgebenden Rohr fließt. Beim Eintropfen in eine Kühlflüssigkeit definierter Dichte (z.B. Paraffinöl) nehmen die Kapseln aufgrund der Grenzflächenspannung Kugelgestalt an und erstarren. Als Füllgüter eignen sich ölige Trägermaterialien. Das Verfahren liefert nahtlose, runde Kapseln in einer Stundenleistung bis zu 70.000 Stück. Dieses Tropf- oder Blasverfahren besitzt vor allem die folgenden Nachteile:
a) Es können nur ölige Lösungen als Füllgut verarbeitet werden.
b) Die verschiedenen verfahrenstechnisch bedingten Komponenten, wie ölige Füllung, Gelatinemasse und gekühltes Fällbad (Paraffinöl) lassen sich nur äußerst schwierig aufeinander abstimmen, da es sich hierbei um ein 3-Phasensystem handelt.
c) Die an den weichgelatinekapseln haftenden Reste des Fällbades (Paraffinöl) müssen mit einem Lösungsmittel entfernt werden. Es treten dabei dieselben Schwierigkeiten, wie beim Rotary-Die- bzw. Stanzverfahren unter f) angeführt, auf.

Daher bereiten die nach dem Stand der Technik bekannten Verfahren zur Herstellung von Weichgelatinekapseln technologische und wirtschaftliche Probleme. Der komplexe verfahrenstechnische Aufwand erlaubt den Arzneimittelherstellerfirmen nur unter großen Schwierigkeiten die Installierung und den Betrieb von eigenen Produktionsanlagen für Weichgelatinekapseln. Zusätzliche Probleme können durch mangelhafte Kenntnis über die Eigenschaften von Gelatine auftreten. Weiterhin treten Probleme bei der Reinigung der Kapseln von anhaftendem Trennöl bzw. Kühlöl auf, wozu beim Scherer-Verfahren noch die Entsorgung der Netzabfälle bewältigt werden muß.

Der Erfindung liegt daher die Aufgabe zugrunde, die genannten, im Stand der Technik auftretenden Schwierigkeiten bei der Herstellung von Weichgelatinekapseln zu vermeiden, insbesondere ein technologisch und ökonomisch relativ einfaches Verfahren zur Verfügung zu stellen, das einer Arzneimittelherstellerfirma ohne großen Aufwand die Anschaffung und den Betrieb einer eigenen Produktionsanlage für Weichgelatinekapseln ermöglicht.

Diese Aufgabe wird erfindungsgemäß dadurch gelöst, daß man bei einem verfahren der eingangs genannten Art als Kühlbad tiefkalte Flüssigkeiten wie z.B. flüssigen Stickstoff verwendet.

Insbesondere stellt die vorliegende Erfindung ein Verfahren zur Herstellung von Weichgelatinekapseln nach einem Tropfverfahren zur Verfügung, wobei man das Füllgut mit einer Weichgelatinemasse umhüllt und zur Verfestigung der Gelatinemasse in ein Kühlbad einbringt, dadurch gekennzeichnet, daß man zur Formgebung ein Kühlbad, welches eine tiefkalte Flüssigkeit enthält, die auf der Weichgelatinekapsel keinen biologisch bedenklichen oder schädlichen Rückstand hinterläßt, verwendet.

Dabei werden für das erfindungsgemäße Verfahren Zweistoffdüsen, beispielsweise konzentrische Doppelkapillaren wie sie vom Globex-Verfahren oder von der Kapselmaschine "Spherex" der Firma Freund bekannt sind, eingesetzt. Das erwärmte Hüllmaterial (Weichgelatinemasse) fließt dabei in der äußeren, das Füllmaterial in der inneren Kapillare. Das Abschnüren der Kapseln kann dabei, wenn nötig, stoßweise getaktet oder intermittierend gesteuert werden.

Es zeigt:

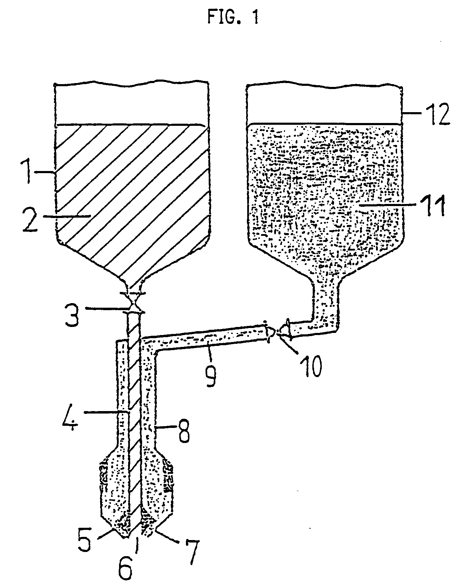

- Fig. 1: eine Schnittansicht einer für das erfindungsgemäße Verfahren verwendeten Dosiervorrichtung

In Fig. 1 wird eine Dosiervorrichtung mit Zweistoffdüse schematisch dargestellt. In dem beheizbaren Gelatinebehälter 12 befindet sich das Kapselwandmaterial 11. Der zweite Behälter 1 enthält das Füllgut 2. Über die regelbare Dosiereinrichtung 3 und 10 kann die Zufuhrmenge gesteuert werden. Hüll- und Füllmaterial fließen über die Zuleitungsrohre 9 und 4 in die Düse 7, wobei die Gelatinelösung über ein Mantelrohr 8 so geführt wird, daß sie über den verstellbaren Düsenmantel 5 das aus dem inneren Düsenkopf 6 austretende Füllgut umhüllen kann. Durch eine intermittierende oder stoßweise getaktete Zuflußregulierung wird die Bildung von Kapseln bewirkt.

Danach werden die Kapseln in einen isolierten Tauchfroster, der mit einem tiefkalten verflüssigten Gas in einem Bereich von -70°C bis -220°C, wie z.B. Flüssigstickstoff, gefüllt ist, eingebracht. Hier geschieht der eigentliche formgebende Prozeß. Durch den extremen Temperaturunterschied zwischen dem erwärmten Gelatine-Hüllmaterial und dem flüssigen Gas verdampft dieses sofort und umgibt die Kapsel mit einem Gaspolster, das einen gleichmäßigen Druck auf die Kapsel ausübt. Die Kapsel nimmt daher eine Kugelgestalt an und erstarrt.

Die so geformten, nahtlosen Kapseln werden aus dem Tauchbad mittels eines Transportbandes heraustransportiert und nach dem Auftauen in an sich bekannter Weise direkt, d.h. ohne die bei üblichen verfahren notwendigen Reinigungsschritte mit organischen Lösungsmitteln, getrocknet.

Gemäß einer besonderen Ausführungsform enthält auch die Gelatinehülle Arzneistoff. Vorzugsweise enthält die Gelatinehülle einen anderen Arzneistoff als das Füllgut, was sich zum Beispiel vorteilhaft zur Vermeidung von Inkompatibilitäten zwischen Arzneistoffen ausnutzen läßt.

Ebenso läßt sich durch das Vorliegen eines Arzneistoffes in der Hülle eine Retardformulierung mit Initialdosis entwickeln, wobei sich in der Hülle die Initialdosis befindet, in dem Füllmaterial der Arzneistoff in einer verzögert freisetzenden Form vorliegt.
Weitere bevorzugte Ausführungsformen des erfindungsgemäßen Verfahrens sind in den Unteransprüchen genannt und beansprucht.

Ein Vorteil des beanspruchten Verfahrens gegenüber dem Rotary-Die- bzw. Stanz-Verfahren besteht darin, daß Gelatinen mit Bloomzahlen von 50 bis 300 verwendet werden können.

Es kommen Gelatinen, fraktionierte Gelatine oder Kollagen zum Einsatz.

Zur Herstellung der Weichgelatinemasse können alle bekannten Weichmacher, wie z.B. Glycerin, Sorbit etc. in einem Bereich von 0,1 - 50% (bezogen auf die Rezepturmasse) sowie alle für Kapselhüllen üblichen Zusatzstoffe (Farbstoffe, Opakisierungsmittel, Pigmente, Aromatisierungs- und Süßmittel, Konservierungsmittel usw.) in einem Bereich von 0,1 - 30% (bezogen auf die Rezepturmasse) eingesetzt werden.

Die Weichgelatinekapseln können mit magensaftresistenten Stoffen in einem anschließenden Überzugsverfahren in bekannter Weise magensaftresistent überzogen werden.

Erfindungsgemäß können Stoffe aus der Gruppe der Polyacrylsäuren und ihren Mischpolymerisaten, Polymethacrylsäuren und ihren Mischpolymerisaten, Celluloseacetatphthalat Hydroxypropylmethylcellulosephtalat, azo-vernetzte Polymethacrylate; Polyurethan-Zucker-Copolymere, wobei als Zuckerkomponente insbesondere oligomere Galactomannane oder Galactomannanderivate geeignet sind, die dann mit aliphatischen Diisocyanaten vernetzt werden; Galactomannanderivate wie Ethyl- oder Acetylgalactomannane; mit Adipinsäure vernetzte Polysaccharide im Bereich von ca. 1 - 20%, bezogen auf die Rezepturmasse, in gelöster Form, z.B. in Form ihrer wasserlöslichen Salze, direkt in die Weichgelatinemasse eingearbeitet werden. Eine solche Vorgehensweise führt zu einem Produkt, das den entsprechenden Vorschriften der Arzneibücher (DAB, USP) standhält, z.B. erreicht man dadurch Magensaftresistenz.

Eine kontrollierte Freigabe kann vorteilhafterweise auch dadurch erfolgen, daß man als Füllgut z.B. Kollagenhydrolysat oder Gelatinen verwendet, denen man pharmazeutisch akzeptable Vernetzungsmittel, wie z.B. Aldosen (Xylose) oder Zitral zusetzt, die eine steuerbare Vernetzung der Füllmatrix ermöglichen. Auf diese Weise können Retardarzneiformen mit unterschiedlicher Freigabecharakteristik des Wirkstoffes realisiert werden.

Als Füllmaterialien sind zunächst grundsätzlich alle in klassisch hergestellten Weichgelatinekapseln umhüllten Arzneistoffe bzw. Füllrezepturen einsetzbar. Dies sind vor allem flüssige Füllgüter wie z.B. ätherische Öle (Pinen, Myrtol Pfefferminzöl etc.), ölige Substanzen wie Vitamin E oder Lebertran, Knoblauchöl, Omega-3-Fettsäuren, Nachtkerzenöl aus Oenothera biennis, Wacholderöl, Johanniskrautöl, Weizenkeimöl, Lecithin etc. Diese lipophilen Stoffe lassen sich auch in Form von Mikroemulsionen in einer entsprechenden Rezepturmasse einsetzen.

Weiterhin sind als Füllmaterialien auch Dispersionen von Arzneistoffen in Kollagenhydrolysat- oder Gelatinemassen geeignet.

Als besonders feindisperse Arzneistoffverteilungen sind ebenso kolloiddisperse Arzneistoffsysteme (Nanosole) geeignet, deren Eigenschaften und Herstellung in zahlreichen Patentanmeldungen Anmeldungen der ALFATEC-Pharma GmbH beschrieben sind (z.B. PCT-Anmeldung PCT/DE92/01010 und dort zitierte weitere PCT-Anmeldungen).

Der erfindungsgemäße Verkapselungsprozeß gestattet ebenfalls die Verwendung von Mikrokapseln oder Koazervaten.

Die Verarbeitung von Arzneistoffen, die im Füllgut gelöst werden können (wasserlösliche Arzneistoffe) läßt sich erfindungsgemäß durch ein Füllgut aus Kollagenhydrolysaten bzw. Gelatinen realisieren, das einen Weichmacherzusatz in der gleichen Konzentration wie die Kapselwand enthält. Auf diese Weise kann eine Diffusion des in der Kapselwand enthaltenen Weichmachers in das Füllgut vermieden werden.

Als Weichmacher können solche dienen, die ausgewählt sind aus der Gruppe bestehend aus: Glycerol, Propylenglykol, Polyethylenglykol, Triacetin, Sorbitol, Sorbitan-Gemische sowie deren Mischungen.

Schwer wasserlösliche Arzneistoffe können in bekannter Form, z.B. lösungsvermittelt, in dem Füllmaterial vorliegen.

Gegenüber dem Tropf- oder Blasverfahren besteht der Vorteil, daß nicht nur ölige, sondern auch pastöse und leichtflüssige Lösungen als Füllgut (ölige Suspensionen von Pflanzenextrakten, Rutin, Beta-Carotin, Mineralstoffe, Vit. ACE-Kombinationen usw.) oder Füllungen, die bei Raumtemperatur erstarren (vorzugsweise Weichgelatinemassen) bzw. halbfest sind, verarbeitet werden können. Wegen des schockartigen Tauchfrostens der Kombination aus Füllgut und dieses umhüllende Gelatinelösung brauchen die rheologischen Eigenschaften sowie die unterschiedlichen Dichten der drei Phasen Füllgut-Gelatinelösung-Tauchbad nicht besonders aufeinander abgestimmt zu werden.

Weitere Ausführungen hierzu sind in den im folgenden aufgelisteten parallelen internationalen (PCT)-Anmeldungen enthalten. Die Inhalte dieser parallelen PCT-Anmeldungen, am selben Tage beim Deutschen Patentamt von denselben Erfindern und Anmeldern eingereicht:
PCT/DE93/00037 entsprechend WO 93/13754
PCT/DE93/00038 entsprechend WO 93/13757
PCT/DE93/00036 entsprechend WO 93/13753
werden hiermit ebenso vollinhaltlich zur Offenbarung der vorliegenden Anmeldung gemacht, wie die älteren PCT-Anmeldungen:
PCT/DE92/01010, PCT/DE92/01012, PCT/DE92/01014, PCT/DE92/01016, PCT/DE92/01007, PCT/DE92/01008, PCT/DE92/01015, PCT/DE92/01013, PCT/DE92/01009, PCT/DE92/01011 vom 4.12.1992.

So werden in der WO 93/13753 (PCT/DE93/00036) auf Seite 7 unter "Formkörper" sowohl Formkörper mit einer Größe von bis zu ca. 2 mm als auch solche mit einer Größe bis zu 12 mm verstanden. Mikroverkapselte Arzneistoffe werden auf Seite 19 erwähnt.

So ist es im Gegensatz zum Globex-Verfahren durch die vorliegende Erfindung möglich, beispielweise Rezepturen auf Basis von kaltwasserlöslichen Gelatinesorten oder Gelatine als Füllmaterial zu verkapseln. Ungeachtet der unterschiedlichen Dichten des Dreiphasensystems bildet sich durch das Tauchfrostverfahren die Kugelgestalt der Kapseln aus. Wenn z.B. gleiche Weichmachermengen dem Hüll- als auch dem gelatinehaltigen Füllmaterial zugesetzt werden, so stellt sich beim Trocknen eine konstante Restfeuchte der gesamten Kapsel ein.

Die oben genannten "festen" Füllungen mit hydrophilen Trägermaterialien in Kapseln bieten die Möglichkeit, den Arzneistoff verzögert freizusetzen. So kann beispielsweise unter Verwendung von speziellen Gelatinesorten für Füllrezepturen, die einen Erweichungspunkt oberhalb 37°C besitzen, eine Matrix für den Arzneistoff formuliert werden, deren Abbau zeitgesteuert erfolgt. Die genannten "festen" Füllungen sind weiterhin geeignet, stabile Emulsionen, in Wasser gelöste Arzneistoffe bzw. Mikrokapseln zu enthalten.

Des weiteren können zu üblichen Weichgelatinemassen Zusätze, insbesondere polymere Makromoleküle zur Steuerung der Arzneistofffreisetzung (controlled released) der Füllmasse zugesetzt werden:
Zum Beispiel können Alginate, Pektine, thermoreversible Alginatgele, Agar-Agar, Albumine, Kasein, Pflanzenproteine, Gummi arabicum, Xanthan, Tragant, Chitosan, Polyethylenglykol, natürliche und modifizierte Stärken, Maltodextrin, Methylcellulose, Celluloseäther-polysaccharide, Carboxymethylcellulosen, verätherte Carboxymethylcellulose, Hydroxypropylcellulose, Hydroxypropylmethylcellulosephtalat, Celluloseacetatphtalat, Polyvinylalkohol, Polyvinylpyrrolidon, Polyacrylsäure, Mischpolymere aus Methacrylsäure und Methacrylsäureestern, Aldosen wie Xylose, Citral in unterschiedlichen Anteilen und Mischungen untereinander zum Einsatz kommen.

Weiterhin ist zur Retardierung bzw. gesteuerten Arzneistofffreigabe aus den erfindungsgemäßen Gelatinekapseln vorteilhaft auch eine kontrollierte Vernetzung der Gelatinehülle durchführbar. Hierfür sind insbesondere untoxische Aldehyde wie z.B. Xylose oder andere Aldosen oder Zitral geeignet. Nach Applikation solcher Kapseln mit vernetzten Hüllen bildet sich im physiologischen Milieu eine Diffusionshülle aus. Durch den Grad der Vernetzung der Bestandteile der Kapselhülle, der z.B. über die Menge an zugesetzter xylose beeinflußt werden kann, lassen sich unterschiedlichste Arzneistoff-Freigabeprofile erzielen.

Als tiefkaltes verflüssigtes Gas eignet sich jedes verflüssigtes Gas, in der die Gelatinemasse augenblicklich erstarrt und die keine schädlichen Rückstände auf oder in der Weichgelatinekapsel zurückläßt. Besonders bevorzugt ist flüssiger Stickstoff.

Durch die obengenannte Schockfrostung bildet sich beim schnellen, augenblicklich erzwungenen Übergang der Gelatinemasse vom Solzustand in den Gelzustand ein amorphes Gelatinegerüst aus. Solche Gelatinegerüste zeichnen sich z.B. durch eine schnellere Auflösungszeit aus, im Vergleich zu konventionellen Gelatinekapseln mit gleicher Gelatinequalität.

Weitere tiefkalte Flüssigkeiten, die sich für das erfindungsgemäße Verfahren eignen können, sind z.B. flüssige Gase, z.B. Argon oder flüssige Luft etc.

Somit können mit dem erfindungsgemäßen Verfahren Weichgelatinekapseln hergestellt werden, die sowohl als Arzneimittel, als diätetische Lebensmittel oder in der Kosmetik (z.B. Badeöl enthaltende Kapseln oder Wirkstoff-Konzentrat-Kapseln) verwendet werden können.

Weiterhin entfällt bei der erfindungsgemäßen Herstellung die Verwendung von teuren und störungsanfälligen Präzisions-Formwalzen. Die Technik ist so einfach, daß nur ein geringer apparativer und verfahrensmäßiger Aufwand notwendig und das erforderliche Know-how wesentlich einfacher zu erlangen ist. Es fallen keine großen Mengen von Gelatineabfällen an. Die aufwendige Klimatisierung der Herstellungsräume kann entfallen. Die entstandene Gelatinekapsel ist nahtlos. Durch Aneinanderreihen mehrerer Herstellungsgeräte läßt sich die Stundenleistung nahezu beliebig erhöhen.

Als wesentlicher Vorteil entfällt beim erfindungsgemäßen Verfahren die Reinigung der fertigen Weichgelatinekapseln von anhaftendem Öl (Trennöl zwischen Walzen und Gelatinebändern oder Paraffinöl aus dem Fällbad), die bei allen bekannten Verfahren nach dem Stand der Technik notwendig ist. Deshalb kann der hiermit nach dem Stand der Technik notwendige apparative Aufwand eingespart werden. Die Problematik, die sich bei der Behandlung von arzneistoffenthaltenden Weichgelatinekapseln mit gesundheitsschädlichen und ökologisch bedenklichen Lösungsmitteln und bei der Entfernung der Lösungsmittel aus der Abluft ergeben, entfällt beim vorgeschlagenen Verfahren vollständig. Darüberhinaus sind die eingesetzten geeigneten tiefkalten Flüssigkeiten wie z.B. flüssiger Stickstoff chemisch völlig inert.

### Beispiel 1:

### Rezepturmasse für die Hülle:

| | |
|---|---|
| Gelatine 250 Bloom | 2,5 Teile |
| Glycerol (85%ig) | 1 Teil |
| Wasser | 6,5 Teile |

### Rezepturmasse für die Füllung:

| | |
|---|---|
| D,L-alpha-Tocopherolacetat | 1 Teil |
| Sojabohnenöl | 1 Teil |

Das Gelatinegranulat wird 30 Minuten vorgequollen und anschließend bei 60°C gelöst. Glycerol wird unter Rühren zugegeben. Nach Entgasen im Vakuum wird die erhaltene Masse in den auf 60°C geheizten Vorratsbehälter 11 der in Fig. 1 beschriebenen Zweidüsenvorrichtung überführt. In das ebenfalls auf 60°C geheizte Vorratsbehältnis 2 wird die auf die gleiche Temperatur erwärmte Lösung des Tocopherolacetats in Sojabohnenöl gefüllt. Über das Mantelrohr 8 wird die Gelatinelösung so zugeführt, daß sie über den Düsenmantel 5 das aus dem inneren Düsenkopf 6 austretende Füllgut umhüllen kann. Über das Zuleitungsrohr 4 wird das Füllgut zudosiert. Es werden Kapseln getaktet mit einer Dosis von 150 mg Tocopherolacetat pro Kapsel abgeschnürt und in das mit flüssigem Stickstoff gefüllte Tauchbad eingetropft. Sie erstarren sofort zur festen Kugelform und werden über ein Transportband in einen Auffangbehälter gefördert. Nach Auftauen werden sie in an sich bekannter Weise auf eine Restfeuchte von 7 bis 8% aufgetrocknet. (1-2 h Trockentumbler Vortrocknung, dann im Umlufttrockner auf 7-8 % Restfeuchte aufgetrocknet.

### Beispiel 2:

### Bestandteile der Kapselhülle:

Gelatine 160 Bloom 30 T
Glycerol 15 T
Eisenoxid 0,1 T
Wasser 54,9 T
Das Kapselhüllmaterial wird wie in Beispiel 1 hergestellt, wobei das Eisenoxid in der Lösung suspendiert wird. Für das Füllmaterial wird das Polyethylenglykol 4000 mit Polyethylenglykol 400 vermischt und auf 60°C erwärmt. Aerosil und der Wirkstoff werden darin suspendiert. Beide Mischungen werden in die jeweiligen Vorratsbehälter gefüllt, die auf 60°C geheizt werden.

### Bestandteile der Fülle:

Polyethylenglykol 400 90 T
Polyethylenglykol 4000 2 T
Aerosil:8 T
25 mg Indometacin
Der Arzneistoff wird mit der Trägermasse homogen vermischt und analog Beispiel 1 in einem Flüssigstickstoffbad zu Kapseln mit einem Gehalt von 25 mg Indometacin geformt.

## Patentansprüche

1. Verfahren zur Herstellung von Weichgelatinekapseln nach einem Tropfverfahren, wobei man das Füllgut mit einer Weichgelatinemasse umhüllt und zur Verfestigung der Gelatinemasse in ein Kühlbad einbringt, dadurch gekennzeichnet, daß man zur Formgebung ein Kühlbad verwendet, welches ein tiefkaltes verflüssigtes Gas enthält, das auf der Weichgelatinekapsel keinen biologisch bedenklichen oder schädlichen Rückstand hinterläßt.

2. Verfahren nach Anspruch 1, dadurch gekennzeichnet, daß man ein verflüssigtes Gas mit einem Siedebereich von -70°C bis -220°C einsetzt.

3. Verfahren nach Anspruch 2, dadurch gekennzeichnet, daß man als verflüssigtes Gas flüssige Luft einsetzt.

4. Verfahren nach Anspruch 2, dadurch gekennzeichnet, daß man flüssigen Stickstoff einsetzt.

5. Verfahren nach einem der Ansprüche 1-4, dadurch gekennzeichnet, daß das Füllgut ausgewählt wird aus der Gruppe bestehend aus: Arzneistoffe, Wirkstoffe, diätetische Lebensmittel, Kosmetika, flüssige Stoffe, pastöse Stoffe, Stoffe mit suspendierten Bestandteilen, Emulsionen, mikroverkapselte Stoffe; sowie deren Mischungen.

6. Verfahren nach einem der Ansprüche 1-5, dadurch gekennzeichnet, daß man sowohl der Gelatinehülle als auch dem Füllgut oder als Füllgut einen Wirkstoff zusetzt.

7. Verfahren nach Anspruch 6, dadurch gekennzeichnet, daß man dem Füllgut oder als Füllgut einen anderen Wirkstoff zusetzt als man der Gelatinehülle zusetzt.

8. Verfahren nach Anspruch 1, dadurch gekennzeichnet, daß dem Füllgut ein Weichmacher zugesetzt wird, ausgewählt aus der Gruppe bestehend aus: Glycerol, Propylenglykol, Polyethylenglykol, Triacetin, Sorbitol, Sorbitan-Gemische; sowie deren Mischungen.

9. Verfahren nach Anspruch 8, dadurch gekennzeichnet, daß das Füllgut und die Gelatinehülle den Weichmacher in annähernd gleicher Konzentration enthält.

10. Verfahren nach einem der Ansprüche 1-9, dadurch gekennzeichnet, daß man als Gelatine eine Sol-Gel-bildende Gelatine mit einer Bloom-Zahl von ca. 50 bis 300 Bloom einsetzt.

11. Verfahren nach Anspruch 10, dadurch gekennzeichnet, daß man eine Gelatine verwendet ausgewählt aus der Gruppe bestehend aus: fraktionierte Gelatine, Gelatinederivate, Kollagenhydrolysate; sowie deren Mischungen.

12. Verfahren nach Anspruch 1, dadurch gekennzeichnet, daß man dem Füllgut Stoffe zusetzt, welche eine kontrollierte Freigabe (controlled release) aus dem Füllgut ermöglichen, wobei die Stoffe ausgewählt werden aus der Gruppe bestehend aus: Poly- und Methacrylsäurederivate, Alginate, Aldosen, Citral, Cellulosederivate, Polyethylenglykol, Polyvinylalkohol, Polyvinylpyrrolidon, Agar-Agar, Pektine; sowie deren Mischungen,Hydroxypropylmethylcellulosephtalat, azo-vernetzte Polymethacrylate, Polyurethan-Zucker-Copolymere, wobei als Zuckerkomponente insbesondere oligomere Galactomannane oder Galactomannanderivate geeignet sind, die dann mit aliphatischen Diisocyanaten vernetzt werden, Galactomannanderivate wie Ethyl- oder Acetylgalactomannane, mit Adipinsäure vernetzte Polysaccharide.

13. Verfahren nach einem der Ansprüche 8-12, dadurch gekennzeichnet, daß man als Füllgut einen Arzneistoff einsetzt.

14. Verfahren nach einem der Ansprüche 8-12, dadurch gekennzeichnet, daß man als Füllgut ein diätetisches Lebensmittel einsetzt.

15. Verfahren nach einem der Ansprüche 8-12, dadurch gekennzeichnet, daß man als Füllgut ein Kosmetikum einsetzt.

16. Verfahren nach einem der Ansprüche 1-15, dadurch gekennzeichnet, daß man die Weichgelatinemasse mit einem üblichen magensaftresistenten Stoff mischt und/oder die Weichgelatinekapsel mit einem magensaftresistenten Überzug versieht.

## Claims

1. Process for the production of soft gelatin capsules by a drip-feed process, the filling material being covered with a soft gelatin composition and being introduced into a cooling bath to solidify the gelatin composition, characterized in that, for shaping, a cooling bath is used which contains a deep-cooled liquefied gas which does not leave behind any biologically unacceptable or harmful residue on the soft gelatin capsule.

2. Process according to Claim 1, characterized in that a liquefied gas having a boiling range from -70°C to -220°C is employed.

3. Process according to Claim 2, characterized in that the liquefied gas employed is liquid air.

4. Process according to Claim 2, characterized in that liquid nitrogen is employed.

5. Process according to one of Claims 1-4, characterized in that the filling material is selected from the group consisting of: pharmaceutical substances, active compounds, dietetic foodstuffs, cosmetics, liquid substances, pasty substances, substances containing suspended constituents, emulsions, microencapsulated substances; and their mixtures.

6. Process according to one of Claims 1-5, characterized in that an active compound is added both to the gelatin shell and to the filling material or as filling material.

7. Process according to Claim 6, characterized in that a different active compound is added to the filling material or as filling material than is added to the gelatin shell.

8. Process according to Claim 1, characterized in that a plasticizer is added to the filling material, selected from the group consisting of: glycerol, propylene glycol, polyethylene glycol, triacetin, sorbitol, sorbitan mixtures; and their mixtures.

9. Process according to Claim 8, characterized in that the filling material and the gelatin shell contain the plasticizer in approximately equal concentrations.

10. Process according to one of Claims 1-9, characterized in that the gelatin employed is a sol/gel-forming gelatin having a Bloom number of about 50 to 300 Bloom.

11. Process according to Claim 10, characterized in that a gelatin is used selected from the group consisting of: fractionated gelatin, gelatin derivatives, collagen hydrolysates; and their mixtures.

12. Process according to Claim 1, characterized in that substances are added to the filling material which make possible controlled release from the filling material, the substances being selected from the group consisting of: poly- and methacrylic acid derivatives, alginates, aldoses, citral, cellulose derivatives, polyethylene glycol, polyvinyl alcohol, polyvinylpyrrolidone, agar-agar, pectins; and their mixtures, hydroxypropylmethylcellulose phthalate, azo-crosslinked polymethacrylates, polyurethane/sugar copolymers, a suitable sugar component in particular being oligomeric galactomannans or galactomannan derivatives which are then crosslinked with aliphatic diisocyanates, galactomannan derivatives such as ethyl- or acetylgalactomannans and polysaccharides crosslinked with adipic acid.

13. Process according to one of Claims 8-12, characterized in that the filling material employed is a pharmaceutical substance.

14. Process according to one of Claims 8-12, characterized in that the filling material employed is a dietetic foodstuff.

15. Process according to one of Claims 8-12, characterized in that the filling material employed is a cosmetic.

16. Process according to one of Claims 1-15, characterized in that the soft gelatin composition is mixed with a customary gastric juice-resistant substance and/or the soft gelatin capsule is provided with an enteric coating.

## Revendications

1. Procédé de production de capsules de gélatine molle selon une méthode de coulée en gouttes, dans lequel la charge est enveloppée d'une masse de gélatine molle et introduite dans un bain de refroidissement en vue de solidifier la masse de gélatine, caractérisé en ce qu'on utilise pour donner la forme, un bain de refroidissement qui contient un gaz liquéfié à basse température, qui ne laisse sur la capsule de gélatine molle aucun résidu biologiquement dangereux ou nuisible.

2. Procédé suivant la revendication 1, caractérisé en ce qu'on utilise un gaz liquéfié ayant une plage d'ébullition de -70°C à -220°C.

3. Procédé suivant la revendication 2, caractérisé en ce qu'on utilise comme gaz liquéfié de l'air liquide.

4. Procédé suivant la revendication 2, caractérisé en ce qu'on utilise l'azote liquide.

5. Procédé suivant l'une des revendications 1 à 4, caractérisé en ce qu'on choisit la charge dans le groupe consistant en : médicaments, substances actives, aliments diététiques, cosmétiques, substances liquides, substances pâteuses, substances contenant des constituants en suspension, émulsions, substances microencapsulées ; ainsi que leurs mélanges.

6. Procédé suivant l'une des revendications 1 à 5, caractérisé en ce qu'on ajoute une substance active aussi bien à l'enveloppe de gélatine qu'à la charge ou comme charge.

7. Procédé suivant la revendication 6, caractérisé en ce qu'on ajoute à la charge ou comme charge une substance active autre que celle que l'on ajoute à l'enveloppe de gélatine.

8. Procédé suivant la revendication 1, caractérisé en ce qu'on ajoute à la charge un émollient choisi dans le groupe comprenant le glycérol, le propylèneglycol, un polyéthylèneglycol, la triacétine, le sorbitol, des mélanges de sorbitanne ; ainsi que des mélanges de ces substances.

9. Procédé suivant la revendication 8, caractérisé en ce que la charge et l'enveloppe de gélatine contiennent l'émollient approximativement à la même concentration.

10. Procédé suivant l'une des revendications 1 à 9, caractérisé en ce qu'on utilise comme gélatine une gélatine formant un gel de solution colloïdale ayant un degré Bloom d'environ 50 à 300.

11. Procédé suivant la revendication 10, caractérisé en ce qu'on utilise une gélatine choisie dans le groupe comprenant une gélatine fractionnée, des dérivés de gélatine, des hydrolysats de collagène ; ainsi que des mélanges de ces gélatines.

12. Procédé suivant la revendication 1, caractérisé en ce qu'on ajoute à la charge des substances qui permettent une libération contrôlée, (controlled release) de charge, ces substances étant choisies dans le groupe comprenant : des dérivés d'oxyde polyacrylique et d'acide méthacrylique, des alginates, des aldoses, du citral, des dérivés de cellulose, un polyéthylèneglycol, un polymère d'alcool vinylique, une polyvinylpyrrolidone, de l'agar-agar, des pectines ; ainsi que des mélanges de ces substances, du phtalate d'hydroxypropylméthylcellulose, des polyméthacrylates azo-réticulés, des copolymères polyuréthanne-sucre, auquel cas on peut utiliser comme composants sucre en particulier des galactomannanes ou des dérivés de galactomannanes oligomères, qui sont ensuite réticulés avec des diisocyanates aliphatiques, des dérivés de galactomannanes tels que des éthyl- ou acétylgalactomannanes, des polysaccharides réticulés avec de l'acide adipique.

13. Procédé suivant l'une des revendications 8 à 12, caractérisé en ce qu'on utilise un médicament comme charge.

14. Procédé suivant l'une des revendications 8 à 12, caractérisé en ce qu'on utilise comme charge un aliment diététique.

15. Procédé suivant l'une des revendications 8 à 12, caractérisé en ce qu'on utilise comme charge un cosmétique.

16. Procédé suivant l'une des revendications 1 à 15, caractérisé en ce qu'on mélange la masse de gélatine molle avec une substance classique résistant au suc gastrique et/ou on munit d'un revêtement résistant au suc gastrique les capsules de gélatine molle.
